# FASCICULE DE BREVET EUROPEEN

(11) **EP 2 694 022 B1**
(45) Date de publication et mention de la délivrance du brevet: **28.09.2016**
(21) Numéro de dépôt: 12718698.9
(22) Date de dépôt: 05.04.2012
(51) Int. Cl.: A61K 8/365, A61K 8/81, A61Q 19/00, A61K 8/04, A61K 8/60, A61K 8/06

(54) **COMPOSITION COSMÉTIQUE COMPRENANT UN COMPOSÉ D'ACIDE CUCURBIQUE ET UN MÉLANGE DE POLYMÈRES SULFONIQUE ET ACRYLIQUE**
KOSMETISCHE ZUSAMMENSETZUNG MIT EINER CUCURBINSÄUREVERBINDUNG UND EINER MISCHUNG AUS SULFON-UND ACRYLPOLYMEREN
COSMETIC COMPOSITION INCLUDING A CUCURBIC ACID COMPOUND AND A MIXTURE OF SULFONIC AND ACRYLIC POLYMERS

(30) Priorité: 05.04.2011 FR 1152903; 05.04.2011 FR 1152904; 11.04.2011 US 201161473901 P; 11.04.2011 US 201161473904 P
(43) Date de publication de la demande: 12.02.2014
(73) Titulaire: L'OREAL, 75008 Paris (FR)
(72) Inventeur: ALLEMAND, Sophie, F-75010 Paris (FR); DEVIE, Marie, F-92160 Antony (FR); LEVY, Florence, F-75011 Paris (FR)
(74) Mandataire: Le Blainvaux Bellegarde, Françoise
(86) Numéro de dépôt international: PCT/FR2012/050753
(87) Numéro de publication internationale: WO 2012/143645

(56) Documents cités:
- EP-A1- 0 815 828
- EP-A1- 0 987 014
- EP-A1- 1 331 000
- EP-A2- 2 223 680
- WO-A1-95/13863
- WO-A2-2007/054824
- DE-A1- 10 107 240
- US-A1- 2004 228 824
- US-A1- 2008 050 333

## Description

La présente invention concerne des compositions notamment cosmétiques comprenant un composé d'acide cucurbique, un mélange de polymères sulfonique et acrylique, ainsi que l'utilisation de ces compositions dans un procédé de traitement des matières kératiniques d'êtres humains.

Plus particulièrement, les compositions de l'invention sont destinées au soin et/ou au maquillage des matières kératiniques.

Au sens de l'invention, on entend désigner par « matières kératiniques », par exemple, la peau, les muqueuses, les lèvres, le cuir chevelu, les cils, les sourcils et les cheveux.

Il est connu dans la demande EP-A-1333021 des composés hydrogénés d'acide cucurbique comme l'acide 3-hydroxy-2-pentyl-cyclopentane acétique pour favoriser la desquamation de la peau et stimuler le renouvellement épidermique, lutter contre les signes du vieillissement cutané, améliorer l'éclat du teint et/ou lisser la peau du visage. Dans la demande FR-A62921255 ces composés sont également décrits pour leur utilisation comme agent dépigmentant.

EP2223680 décrit à l'exemple 5 une émulsion comprenant 2% de dérivé d'acide cucurbique, une gomme de xanthane et un homopolymère d'acide 2-acrylamido-2-méthylpropane sulfonique. Ce document ne décrit pas d'association avec un homopolymère d'acide acrylique réticulé.

Toutefois, l'introduction des composés hydrogénés d'acide cucurbique précédemment cités dans une formulation cosmétique aqueuse peut se traduire par une diminution non négligeable de la viscosité, induisant ainsi une fluidification importante de la composition et en conséquence une déstabilisation de la composition.

Une composition trop fluide est difficile à appliquer sur les matières kératiniques. Une telle composition s'écoule des matières kératiniques, notamment de la peau, sur lesquelles elle est appliquée. Son application sur les matières kératiniques que l'on souhaite traiter manque de précision et rend ainsi son usage peu attractif.

En outre, la présence d'un composé hydrogéné d'acide cucurbique s'avère affecter le pouvoir épaississant de certains agents gélifiants conventionnels. En particulier, ce composé déstabilise les gels aqueux d'homopolymère d'acide 2-acrylamido-2-méthylpropane-sulfonique en raison d'une chute de viscosité importante.

Ainsi, il existe un besoin de disposer de compositions cosmétiques comprenant un composé d'acide cucurbique et un homopolymère d'un monomère à groupement sulfonique (en particulier un homopolymère d'acide 2-acrylamido-2-méthylpropane-sulfonique) ne présentant pas une fluidification importante spontanément (pas de chute importante de la viscosité).

De manière surprenante, les inventeurs ont observé que l'addition d'un homopolymère d'acide acrylique réticulé tel que décrit ci-après permet d'obtenir une composition dont la viscosité reste stable, sans fluidification importante. En particulier, la composition présente une bonne stabilité dans le temps, notamment après stockage pendant 2 mois à la température ambiante (25 °C).

La présente invention a précisément pour objet de satisfaire à ces besoins. En outre, la composition selon l'invention lors de son application sur les matières kératiniques, et notamment sur la peau, ne présente pas de sensation de collant.

Plus précisément, la présente invention concerne une composition, comprenant, dans un milieu physiologiquement acceptable contenant un milieu aqueux, au moins un composé d'acide cucurbique de formule (I), un homopolymère d'un monomère à groupement sulfonique et un homopolymère d'acide acrylique réticulé tels que décrits ci-après.

La composition selon l'invention est en particulier une composition cosmétique.

La présente invention concerne également un procédé de traitement non thérapeutique de soin ou de maquillage des matières kératiniques comprenant l'application sur lesdites matières kératiniques d'une composition conforme à l'invention. Avantageusement, un tel procédé est destiné au soin ou maquillage de la peau.

Le composé dérivé d'acide cucurbique est un composé choisi parmi ceux répondant à la formule (I) suivante : 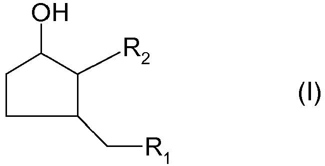 dans laquelle :
R₁ représente un radical COOR₃, R₃ désignant un atome d'hydrogène ou un radical alkyle en C₁-C₄, éventuellement substitué par un ou plusieurs groupes hydroxyle ;
R₂ représente un radical hydrocarboné, saturé ou insaturé, linéaire ayant de 1 à 18 atomes de carbones, ou ramifié ou cyclique ayant de 3 à 18 atomes de carbone ;
ainsi que leurs isomères optiques, et sels correspondants.

De préférence, R₁ désigne un radical choisi parmi -COOH, -COOMe, -COO-CH₂-CH₃, -COO-CH₂-CH(OH)-CH₂OH, -COOCH₂-CH₂-CH₂OH ,-COOCH₂-CH(OH)-CH₃. Préférentiellement, R₁ désigne un radical -COOH.

Préférentiellement, R₂ désigne un radical hydrocarboné, linéaire, saturé ou insaturé, et de préférence ayant de 2 à 7 atomes de carbone. En particulier, R₂ peut être un radical pentyl, pentenyl, hexyle, heptyle.

Selon un mode de réalisation, le composé de formule (I) est choisi parmi l'acide 3-hydroxy-2-[(2Z)-2-pentenyl]-cyclopentane acétique ou l'acide 3-hydroxy-2-pentyl-cyclopentane acétique. De préférence, le composé (I) est l'acide 3-hydroxy-2-pentyl-cyclopentane acétique ; ce composé peut être notamment sous la forme de sel de sodium.

Les sels des composés utilisables selon l'invention sont en particulier choisis parmi les sels de métal alcalin, par exemple sodium, potassium ; les sels de métal alcalino-terreux, par exemple calcium, magnésium, strontium, les sels métalliques, par exemple zinc, aluminium, manganèse, cuivre ; les sels d'ammonium de formule NH₄⁺ ; les sels d'ammonium quaternaires ; les sels d'amines organiques, comme par exemple les sels de méthylamine, de diméthylamine, de triméthylamine, de triéthylamine, d'éthylamine, de 2-hydroxyéthylamine, de bis-(2-hydroxyéthyl)amine, de la tri-(2-hydroxyéthyl)amine ; les sels de lysine, d'arginine. On utilise de préférence les sels choisis parmi les sels de sodium, potassium, magnésium, strontium, cuivre, manganèse, zinc. Préférentiellement, on utilise le sel de sodium.

Le composé de formule (I) défini précédemment peut être présent dans la composition selon l'invention en une teneur allant de 1 à 10 % en poids, par rapport au poids total de la composition, de préférence de 1,5 % à 5 % en poids.

La composition selon l'invention comprend un homopolymère d'un monomère à groupement sulfonique.
La composition selon l'invention comprend également un polymère comportant au moins un monomère à groupement sulfonique. La présence de ce polymère permet d'obtenir une composition présentant de bonnes propriétés de stabilité.

Les polymères comportant au moins un monomère à groupement sulfonique, utilisés dans la composition de l'invention, sont avantageusement hydrosolubles ou hydrodispersibles ou gonflables dans l'eau. Les polymères utilisés conformément à l'invention sont des homopolymères susceptibles d'être obtenus à partir d'au moins un monomère à insaturation éthylénique et à groupement sulfonique, pouvant être sous forme libre ou partiellement ou totalement neutralisée.

De façon préférentielle, les polymères conformes à l'invention peuvent être neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils sont généralement neutralisés. On entend dans la présente invention par « neutralisés », des polymères totalement ou pratiquement totalement neutralisés, c'est-à-dire neutralisés à au moins 90 %.

Les polymères utilisés dans la composition de l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Ces polymères selon l'invention peuvent être réticulés ou non réticulés.

Les monomères à groupement sulfonique du polymère utilisé dans la composition de l'invention sont choisis notamment parmi l'acide vinylsulfonique, l'acide styrènesulfonique, les acides (méth)acrylamido-(C₁-C₂₂)alkylsulfoniques, les acides N-(C₁-C₂₂)alkyl-(méth)acrylamido-(C₁-C₂₂)-alkylsulfoniques comme l'acide undécyl-acrylamido-méthane-sulfonique, ainsi que leurs formes partiellement ou totalement neutralisées, et leurs mélanges.

Selon un mode préféré de réalisation de l'invention, les monomères à groupement sulfonique sont choisis parmi les acides (méth)acrylamido(C₁-C₂₂)alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide 2-méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique, ainsi que leurs formes partiellement ou totalement neutralisées, et leurs mélanges.

Plus particulièrement, on utilise l'acide 2-acrylamido-2-méthylpropane-sulfonique (AMPS) ainsi que ses formes partiellement ou totalement neutralisées.

Lorsque les polymères sont réticulés, les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl-éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10 % en mole et plus particulièrement de 0,2 à 2 % en mole par rapport au polymère.

L'homopolymère de monomères à groupement sulfonique peut être réticulé avec un ou plusieurs agents de réticulation.

Ces homopolymères sont généralement réticulés et neutralisés, et ils peuvent être obtenus selon le procédé de préparation comprenant les étapes suivantes :
(a) on disperse ou on dissout le monomère tel que l'acide 2-acrylamido 2-méthylpropane sulfonique sous forme libre dans une solution de tertio-butanol ou d'eau et de tertio-butanol ;
(b) on neutralise la solution ou la dispersion de monomère obtenue en (a) par une ou plusieurs bases minérales ou organiques, de préférence l'ammoniaque NH₃, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

Les homopolymères d'AMPS préférés sont en général caractérisés par le fait qu'ils comprennent, distribués de façon aléatoire :
a) de 90 à 99,9% en poids de motifs de formule générale (II) suivante : 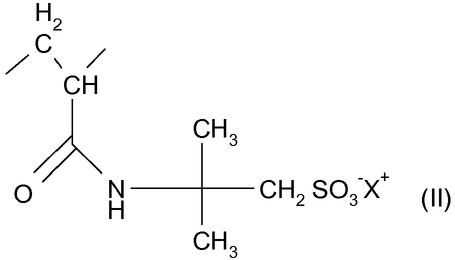 dans laquelle X⁺ désigne un proton, un cation de métal alcalin, un cation alcalino-terreux ou l'ion ammonium, au plus 10% mol des cations X⁺ pouvant être des protons H⁺ ;
b) de 0,01 à 10% en poids de motifs réticulants provenant d'au moins un monomère ayant au moins deux double-liaison oléfiniques ; les proportions en poids étant définis par rapport au poids total du polymère.

Les homopolymères selon l'invention plus particulièrement préférés comprennent de 98 à 99,5 % en poids de motifs de formule (II) et de 0,2 à 2 % en poids de motifs réticulants.

Comme polymères de ce type, on peut citer notamment l'homopolymère réticulé et neutralisé d'acide 2-acrylamido 2-méthylpropane sulfonique, commercialisé par la société Clariant sous la dénomination commerciale « Hostacerin^{®} AMPS » (nom CTFA : ammonium polyacryldimethyltauramide). L'homopolymère de monomère à groupement sulfonique peut être présent dans la composition selon l'invention en une teneur en matière active allant par exemple de 0,05 à 5 % en poids, de préférence allant de 0,1 à 5 % en poids, préférentiellement allant de 0,1 à 2 % en poids, par rapport au poids total de la composition.

Avantageusement, le composé d'acide cucurbique de formule (I) (dit A) et l'homopolymère de monomère à groupement sulfonique (dit B) décrits précédemment peuvent être présents dans la composition selon l'invention dans un rapport pondéral A /B allant de 3 à 4,5

La composition selon l'invention contient un homopolymère d'acide acrylique réticulé.
L'homopolymère peut être réticulé avec un agent réticulant, notamment choisi parmi l'allyl éther de pentaérythritol, l'allyl éther de sucrose, ou l'allyl éther de propylène.

De tels polymères ont pour nom INCI : Carbomer.
On peut utiliser par exemple les polymères vendus par la société Lubrizol sous les dénominations Carbopol 980, 981, Carbopol Ultrez 10 , ou par la société 3V sous la dénomination Synthalen K ou Synthalen L ou Synthalen M.

L'homopolymère d'acide acrylique peut être présent dans la composition selon l'invention en une quantité allant de 0,01 à 5 % en poids, par rapport au poids total de la composition, et de préférence de 0,1 à 3 % en poids.

Avantageusement, le composé d'acide cucurbique de formule (I) (dit A) et l'homopolymère d'acide acrylique (dit C) décrits précédemment peuvent être présents dans la composition selon l'invention dans un rapport pondéral A /C allant de 2 à 20 et de préférence de 4,5 à 5,5.

La viscosité d'une composition de l'invention peut être mesurée selon tout procédé connu de l'homme de l'art, et notamment selon le procédé conventionnel suivant. Ainsi, la mesure peut être réalisée à 25 °C à l'aide d'un Rhéomat 180, équipé d'un mobile tournant à 200t/mn. L'homme du métier peut choisir le mobile permettant de mesure la viscosité, parmi les mobiles, M1 ou M2 ou M3 ou M4 sur la base de ses connaissances générales, de manière à pouvoir réaliser la mesure.

La composition selon l'invention comprend un milieu aqueux physiologiquement acceptable.
Par « milieu physiologique acceptable », on entend désigner un milieu compatible avec les matières et/ou les fibres kératiniques d'êtres humains, comme par exemple, de manière non limitative, la peau, les muqueuses, les ongles, le cuir chevelu et/ou les cheveux.
Ce milieu aqueux physiologiquement acceptable comprend une phase aqueuse, éventuellement en mélange ou non avec un ou plusieurs solvants organiques tels qu'un alcool en C₁-C₈, notamment l'éthanol, l'isopropanol, le tert-butanol, le n-butanol, des polyols comme la glycérine, le propylène glycol, le butylèneglycol, et des éthers de polyol.

Une composition selon l'invention peut également comprendre une phase grasse, qui peut comprendre des huiles, des gommes, des cires usuellement utilisées dans le domaine d'application considéré.

Ainsi selon un mode de réalisation une composition selon l'invention peut en outre comprendre au moins une phase grasse choisie parmi une phase grasse solide à température ambiante (20-25 °C) et pression atmosphérique et/ou une phase grasse liquide à température ambiante (20-25 °C) et pression atmosphérique.

Une phase grasse liquide convenant à la mise en oeuvre de l'invention peut comprendre une huile volatile, une huile non volatile, et un mélange de celles-ci. Une huile volatile ou non volatile peut être une huile hydrocarbonée, notamment d'origine animale ou végétale, une huile synthétique, une huile siliconée, une huile fluorée ou un mélange de celles-ci.

Une phase grasse solide convenant à la mise en oeuvre de l'invention peut être, par exemple, choisie parmi les corps gras pâteux, les gommes, et leurs mélanges.

Comme huiles utilisables dans l'invention, on peut citer les huiles minérales (huile de vaseline), les huiles végétales (fraction liquide du beurre de karité, huile de tournesol), les huiles de synthèse (huile de Purcellin), les huiles ou cires siliconées (cyclométhicone) et les huiles fluorées (perfluoropolyéthers). On peut ajouter à ces huiles des alcools gras et des acides gras (acide stéarique).

Lorsqu'une composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique.

Un ou plusieurs émulsionnants peuvent être présents dans une composition de l'invention en une proportion allant de 0,3 % à 30 % en poids, et en particulier de 0,5 à 20 % en poids par rapport au poids total de la composition.
Lorsque la composition est sous forme d'émulsion huile-dans -eau, elle comprend de préférence un tensioactif de type polyalkylglucoside.

Plus précisément, la présente invention concerne également une composition sous forme d'émulsion huile-dans-eau, comprenant un composé d'acide cucurbique de formule (I), un tensioactif de type alkylpolyglycoside, un homopolymère d'un monomère à groupement sulfonique et un homopolymère d'acide acrylique réticulé tels que décrits ci-après.
Les émulsions obtenues sont particulièrement stables. En particulier, la composition présente une bonne stabilité après stockage pendant 2 mois à 45 °C.
En outre, les compositions selon l'invention sont agréable à appliquer sur les matières kératiniques, notamment sur la peau : elle présente une texture qui se fluidifie lors de l'étalement, passant d'un état fluide épaissi à un état liquide. Cette application se fait sans sensation de collant, ni de peluchage.

La composition selon l'invention comprend au moins un tensioactif de type alkylpolyglycoside.

Au sens de la présente invention, on entend par « alkylpolyglycoside », un alkylmonooside (degré de polymérisation 1) ou alkylpolyoside (degré de polymérisation supérieur à 1).

Les alkylpolyglycosides peuvent être utilisés seuls ou sous forme de mélanges de plusieurs alkylpolyglycosides. Ils répondent en général à la structure suivante :

R(O)(G)ₓ

dans laquelle le radical R est un radical alkyle linéaire ou ramifié en C₁₂-C₂₂, G est un reste de saccharide et x varie de 1 à 5, de préférence de 1,05 à 2,5 et plus préférentiellement de 1,1 à 2.

Le reste saccharide peut être choisi parmi glucose, dextrose, saccharose, fructose, galactose, maltose, maltotriose, lactose, cellobiose, mannose, ribose, dextrane, talose, allose, xylose, levoglucane, cellulose ou amidon. Plus préférentiellement, le reste saccharide désigne glucose.

Il est en outre à noter que chaque unité de la partie polyoside de l'alkylpolyglycoside peut être sous forme isomérique α ou β, sous forme L ou D et la configuration du reste saccharide peut être de type furanoside ou pyranoside.

Il est bien entendu possible d'utiliser des mélanges d'alkylpolyosides, susceptibles de différer les uns des autres par la nature du motif alkyle porté et/ou la nature de la chaîne polyoside porteuse.

Le tensioactif de type alkylpolyglycoside peut être présent dans une composition de l'invention en une teneur allant de 0,1 % à 1,6 % en poids, par rapport au poids total de la composition, de préférence allant de 0,1 % à 1,5 % en poids, et préférentiellement allant de 0,1 % à 1 % en poids.

Selon un mode particulier de l'invention, la composition selon l'invention peut comprendre également au moins un alcool gras, notamment un alcool gras présentant de 10 à 30 atomes de carbone.

A titre d'exemples d'alcools gras pouvant être utilisés, on peut citer les alcools gras linéaires ou ramifiés, d'origine synthétique, ou encore naturelle comme par exemple les alcools provenant de matières végétales (coprah, palmiste, palme, etc) ou animales (suif, etc).

On utilise de préférence un alcool gras comprenant de 10 à 26 atomes de carbone, de préférence de 10 à 24 atomes de carbone, et plus préférentiellement de 12 à 22 atomes de carbone.

A titre d'exemples particuliers d'alcools gras utilisables dans le cadre de la présente invention, on peut notamment citer l'alcool laurique, myristique, cétylique, stéarylique, isostéarylique, palmitique, oléique, cétéarylique (mélange d'alcool cétylique et stéarylique), béhénique, érucique, arachidylique, et leurs mélanges.

En outre, il est particulièrement avantageux, selon la présente invention, de mettre conjointement en oeuvre un alcool gras et un alkylpolyglycoside dont la partie alkyle est identique à celle de l'alcool gras retenu.

Des mélanges émulsionnants alcool gras/alkylpolyglycoside tels que définis sont décrits notamment dans les demandes WO 92/06778, WO 95/13863 et WO 98/47610.

Parmi les mélanges alcool gras/alkylpolyglycoside particulièrement préférés, on peut citer les produits vendus par la société SEPPIC sous les appellations MONTANOV^{®} tels que les mélanges suivants :
- Alcool cétylstéarylique/Cocoglucoside - MONTANOV 82^{®},
- Alcool arachidylique et alcool béhénylique/arachidylglucoside - MONTANOV 802^{®}, ,
- Alcool myristylique/Myristylglucoside - MONTANOV 14^{®},
- Alcool cétylstéarylique/Cétylstéarylglucoside - MONTANOV 68^{®},
- Alcool en C₁₄-C₂₂/C₁₂-C₂₀ alkylglucoside - MONTANOV L^{®},
- Cocoalcool /Coco-glucoside - MONTANOV S^{®}, et
- Alcool isostéarylique/Isostéarylglucoside - MONTANOV WO 18^{®}.

Selon un mode de réalisation particulier, l'alkylpolyglycoside mis en oeuvre dans une composition selon l'invention est le C₁₂-C₂₀ glucoside. Il est avantageusement utilisé en mélange avec un alcool en C₁₄-C₂₂.

Selon un mode de réalisation particulier de l'invention, on utilise ainsi le mélange alcool en C₁₄-C₂₂/C₁₂-C₂₀ alkylglucoside, tel que celui commercialisé par la société SEPPIC sous la dénomination MONTANOV 68^{®}, constitué d'environ 20 % de C₁₂-C₂₀ alkylglucoside et d'environ 80 % d'alcool en C₁₄-C₂₂.

L'alcool gras peut être présent dans une composition de l'invention en une teneur allant de 0,4 % à 8 % en poids, par rapport au poids total de la composition, de préférence allant de 0,1 % à 1 % en poids, et préférentiellement allant de 0,6 % à 2 % en poids.

Le mélange alcool gras/alkylpolyglycoside peut être présent dans une composition de l'invention en une teneur allant de 0,5 % à 8 % en poids, par rapport au poids total de la composition, de préférence allant de 0,6 % à 5 % en poids, et plus préférentiellement de 0,8 % à 2,5 % en poids.

Selon un mode de réalisation particulier, une composition conforme à l'invention peut comprendre ledit composé de formule (I) et ledit alkylpolyglycoside dans un rapport pondéral composé (I) / alkylpolyglycoside allant de 15 à 25, de préférence allant de 17 à 23, et préférentiellement allant de 18 à 22.

Dans le cas particulier où l'alkylpolyglycoside est mis en oeuvre en association avec au moins un alcool gras tel que décrit précédemment, une composition de l'invention peut alors avantageusement comprendre ledit mélange d'alkylpolyglycoside et d'alcool gras et ledit composé de formule (I) dans un rapport pondéral composé (I) / (alkylpolyglycoside + alcool gras) allant de 3 à 5, de préférence allant de 3,4 à 4,6, et préférentiellement allant de 3,6 à 4,4. De préférence, la viscosité des émulsions comprenant un alkylpolyglycoside peut aller de 0,086 Pa.s⁻¹ à 1,4 Pa.s⁻¹. Avantageusement, cette viscosité peut aller de 0,20 Pa.s⁻¹ à 1,1 Pa.s⁻¹.

Avantageusement, le composé d'acide cucurbique de formule (I) (dit A) et l'homopolymère de monomère à groupement sulfonique (dit B) décrits précédemment peuvent être présents dans les émulsions selon l'invention comprenant un tensioactif de type alkylpolyglycoside dans un rapport pondéral A /B allant de 3,5 à 6,5
De préférence, ce rapport pondéral A/B peut aller de 4 à 6. Préférentiellement, ce rapport pondéral A/B peut aller de 4,5 à 5,5.

Avantageusement, le composé d'acide cucurbique de formule (I) (dit A) et l'homopolymère d'acide acrylique (dit C) décrits précédemment peuvent être présents dans les émulsions selon l'invention comprenant un tensioactif de type alkylpolyglycoside dans un rapport pondéral A /C allant de 8 à 12. De préférence, ce rapport pondéral A / C peut aller de 9 à 11. Préférentiellement, ce rapport pondéral A/C peut aller de 9,5 à 10,5.

La composition peut comprendre de l'eau en une teneur allant de 20 % à 95 % en poids, par rapport au poids total de la composition, de préférence allant de 30 % à 90 % en poids, et préférentiellement allant de 40 % à 70 % en poids.

L'eau peut être une eau florale telle que l'eau de bleuet et/ou une eau minérale telle que l'eau de VITTEL, l'eau de LUCAS ou l'eau de LA ROCHE POSAY et/ou une eau thermale.

La composition peut comprendre en outre un solvant organique miscible à l'eau à la température ambiante (25 °C) notamment choisi parmi les monoalcools ayant de 2 à 6 atomes de carbone tels que l'éthanol, l'isopropanol ;
les polyols ayant notamment de 2 à 20 atomes de carbones, de préférence ayant de 2 à 10 atomes de carbone, et préférentiellement ayant de 2 à 6 atomes de carbones, tels que la glycérine, le propylène glycol, le butylène glycol, le pentylène glycol, l'hexylène glycol, le dipropylène glycol, le diéthylène glycol ;
les éthers de glycol (ayant notamment de 3 à 16 atomes de carbone) tels que les alkyl(C₁-C₄)éther de mono, di- ou tripropylène glycol, les alkyl(C₁-C₄)éthers de mono, di- ou triéthylène glycol ;
et leurs mélanges.

La composition selon l'invention peut comprendre un solvant organique miscible à l'eau à la température ambiante, notamment un polyol, en une teneur allant de 1 % à 20 % en poids, par rapport au poids total de la composition, et de préférence allant de 3 % à 15 % en poids.

Avantageusement, la composition selon l'invention a un pH allant de 5,5 à 7,5 .

L'émulsion selon l'invention comprend également une phase huileuse.

Comme huiles utilisables dans la composition de l'invention, on peut citer par exemple :
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations « Miglyol 810 », « 812 » et « 818 » par la société Dynamit Nobel, l'huile de jojoba, l'huile de beurre de karité;
- les esters et les éthers de synthèse, notamment d'acides gras, comme les huiles de formules R1COOR2 et R1OR2 dans laquelle R1 représente le reste d'un acide gras comportant de 8 à 29 atomes de carbone, et R2 représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle ; les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;

- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

L'huile peut être présente dans la composition selon l'invention en une teneur allant de 0,5 % à 20 % en poids, par rapport au poids total de la composition, de préférence allant de 1 % à 15 % en poids.

La phase huileuse de l'émulsion peut comprendre d'autres corps gras tels que par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

La composition selon l'invention peut contenir également des adjuvants cosmétiques notamment choisi parmi les émulsionnants, les gélifiants, les huiles, les cires, les conservateurs, les antioxydants, l'eau, les parfums, les charges, les filtres UV, les pigments, les fibres, les agents chélatants, les absorbeurs d'odeur, les matières colorantes.

Les quantités de ces différents adjuvants sont celles classiquement utilisées dans le domaine cosmétique, et peuvent par exemple varier de 0,01 % à 30 % du poids total de la composition. De manière générale, les quantités sont ajustées en fonction de la formulation réalisée. Ces adjuvants, selon leur nature, peuvent être introduits dans la phase grasse, dans la phase aqueuse et/ou dans les sphérules lipidiques.

La composition selon l'invention peut être sous la forme d'une solution aqueuse, hydroalcoolique ; d'une dispersion; d'une émulsion eau-dans-huile, huile-dans-eau ou multiple; d'une suspension; de microcapsules ou microparticules ; de dispersions vésiculaires de type ionique et/ou non ionique ; de composition pour aérosol comprenant également un agent propulseur sous pression. Préférentiellement, la composition selon l'invention peut être une émulsion huile-dans-eau ou eau-dans-huile. Plus préférentiellement, la composition selon l'invention est une émulsion huile-dans-eau.

Lorsque la composition comprend une phase huileuse, cette dernière peut comprendre un élastomère de silicone. Des exemples d'élastomères de silicone sont décrits dans la demande WO-A-2009/080958.

Une composition selon l'invention peut également se présenter sous la forme d'un produit de soin, d'un produit solaire ou après solaire, d'un produit de soin de photo-protection quotidienne, d'un produit pour le corps, d'un fond de teint à appliquer sur le visage ou sur le cou, d'un produit anti-cernes, d'un correcteur de teint, d'une crème teintée ou d'une base de maquillage pour le maquillage pour le visage ou d'une composition de maquillage pour le corps.

Une composition selon l'invention peut être mise en oeuvre à des fins d'amélioration de l'état général d'un épiderme, en particulier de la peau, et notamment pour le maintient ou la restauration de ses fonctions physiologiques et/ou de son aspect esthétique.

D'autres caractéristiques et avantages de l'invention ressortiront mieux des exemples qui suivent, donnés à titre illustratif et non limitatif. Dans ce qui suit ou ce qui précède, les proportions sont données en pourcentage pondéral, sauf indications contraires.

### Exemples 1 et 2 comparatifs :

On a réalisé un gel aqueux (ex 1) selon l'invention contenant le mélange de polymères sulfonique et acrylique et un gel aqueux hors invention (ex 2) similaire mais ne contenant que le polymère sulfonique ; chaque gel a été réalisé avec ou en l'absence de sel de sodium de l'acide 3-hydroxy-2-pentyl-cyclopentane acétique.
On a ensuite mesuré la viscosité des gels aqueux obtenus après 24 heures de stockage à température ambiante (viscosité mesurée à 25°C à l'aide d'un Rhéomat 180 mobile M3 après 10 minutes de rotation à 200 tours/minutes). On a obtenu les résultats suivants :

| | **Exemple 1A** | **Exemple 1B (invention)** |
|---|---|---|
| Sel de sodium de l'acide 3-hydroxy-2-pentyl-cyclopentane acétique à 30 % dans un mélange eau/dipropylène glycol (70/30). | 0 | 6,6 % soit 2% MA |
| Acide poly acrylamidométhyl propane sulfonique neutralisé partiellement à l'ammoniaque et hautement réticulé | 0,6 % MA | 0,6 % MA |
| (Hostacerin AMPS^{®} de chez Clariant) | | |
| homopolymère d'acide acrylique réticulé (Synthalen K de chez 3V) | 0,4 % | 0,4 % |
| Eau | Qsp 100 % | Qsp 100 % |
| **Viscosité (Pa.s)** | 4 | 0,15 |

| | **Exemple 2A** | **Exemple 2B TEMOIN** |
|---|---|---|
| Sel de sodium de l'acide 3-hydroxy-2-pentyl-cyclopentane acétique à 30 % dans un mélange eau/dipropylène glycol (70/30) | 0 | 6,6 % soit 2% MA |
| Acide poly acrylamidométhyl propane sulfonique neutralisé partiellement à l'ammoniaque et hautement réticulé (Hostacerin AMPS^{®} de chez Clariant) | 1 % MA | 1 % MA |
| Eau | Qsp 100 % | Qsp 100 % |
| Viscosité (Pa.s) | 4 | 0,038 |

Ces essais montrent que l'association de polymère sulfonique (Hostacerin AMPS) et de polymère acrylique réticulé (Carbomer tel que Synthalen K) en présence du sel de sodium de l'acide 3-hydroxy-2-pentyl-cyclopentane acétique présente une faible variation de viscosité.
Ainsi, la présence du mélange du polymère acrylique permet d'éviter une chute très importante de la viscosité du gel aqueux contenant le polymère sulfonique en présence du sel de sodium de l'acide 3-hydroxy-2-pentyl-cyclopentane acétique.

### Exemple 3 :

On a réalisé une crème de soin de la peau ayant la composition suivante :

| **Exemple** | **3** |
|---|---|
| Sel de sodium de l'acide 3-hydroxy -2-pentyl-cyclopentane acétique à 30 % dans un mélange eau/dipropylène glycol (70/30) | 13,4 % soit 4 % MA |
| homopolymère d'acide acrylique réticulé (CARBOPOL ULTREZ 10 de chez Lubrizol) | 0,25 |
| Acide poly acrylamidométhyl propane sulfonique neutralisé partiellement à l'ammoniaque et hautement réticulé (HOSTACERIN AMPS^{®} de chez Clariant) | 0,5 |
| hydroxyéthyl cellulose (NATROSOL 250 HHR de chez AQUALON) | 0,25 |
| Hyaluronate de sodium (CRISTALHYAL de chez SOLIANCE) | 0,1 |
| Gomme de xanthane | 0,1 |
| Ethanol | 5 |
| Glycérine | 3 |
| Hydroxyde de sodium | 0,12 |
| Sel disodique de l'acide éthylène diamine tétracétique | 0,1 |
| Conservateur | qs |
| Eau | qsp 100 |
| **centrifugation** | Reste homogène |
| **aspect microscopique** | homogène |
| **Stabilité 2 mois à 25 °C et à 45 °C** | stable |

La composition s'étale agréablement sur la peau sans sensation d'effet collant et sans pelucher.

### Exemples 4 à 6 comparatifs

On a réalisé une 3 émulsions huile-dans eau (sérum de soin de la peau) contenant le sel de sodium de l'acide 3-hydroxy-2-pentyl-cyclopentane :
Une émulsion (exemple 4) selon l'invention comprenant l'association de l'acide poly acrylamidométhyl propane sulfonique neutralisé partiellement à l'ammoniaque et réticulé (Hostacerin AMPS^{®} de chez Clariant) (dit polymère B) et de l'homopolymère d'acide acrylique réticulé (Carbopol Ultrez 10 de chez Lubrizol) (dit polymère C) ;

Une émulsion (exemple 5) hors invention similaire à l'exemple 4 dans laquelle la quantité de polymère C a été remplacée par une même quantité de polymère B;
Une émulsion (exemple 6) hors invention similaire à l'exemple 4 dans laquelle la quantité de polymère B a été remplacée par la même quantité de polymère C;

Pour chaque composition, on a effectué une évaluation microscopique de la composition.

On a obtenu les résultats suivants :

| **Exemple** | **4 (invention)** | **5 (hors invention)** | **6 (hors invention)** |
|---|---|---|---|
| Sel de sodium de l'acide 3-hydroxy-2-pentyl-cyclopentan e acétique à 30 % dans un mélange eau/dipropylène glycol (70/30) | 13,4 % soit 4 % MA | 13,4 % soit 4 % MA | 13,4 % soit 4 % MA |
| Eau | Qsp 100 | Qsp 100 | Qsp 100 |
| **homopolymère d'acide acrylique réticulé (CARBOPOL ULTREZ 10 de LUBRIZOL) (polymère C)** | **0,4** | **0** | **1,2** |
| **Acide polyacrylamido méthyl propane sulfonique neutralisé partiellement à l'ammoniaque et réticulé (HOSTACERIN AMPS^{®} de CLARIANT) (polymère B)** | **0,8** | **1,2** | **0** |
| Hydroxyéthyl cellulose (NATROSOL 250 HHR de AQUALON) | 0,25 | 0,25 | 0,25 |
| Hyaluronate de sodium (CRISTALHYAL de SOLIANCE) | 0,1 | 0,1 | 0,1 |
| Glycérol | 8 | 8 | 8 |
| Ethanol | 5 | 5 | 5 |
| Mélange alcool en C₁₄-C₂₂/C₁₂-C₂₀ alkylglucoside (80/20) (MONTANOV 68^{®} de | 1 | 1 | 1 |
| SEPPIC) | | | |
| Polydimethylsiloxane 10 cst | 2 | 2 | 2 |
| Huile de limnanthes alba | 0,5 | 0,5 | 0,5 |
| Huile de vaseline | 1 | 1 | 1 |
| Sel disodique de l'acide éthylène diamine tétracétique | 0,1 | 0,1 | 0,1 |
| Hydroxyde de sodium | 0,2 | 0,07 | 0,46 |
| Adénosine | 0,1 | 0,1 | 0,1 |
| Conservateur | qs | qs | qs |
| | | | |
| **aspect microscopique** | Homogène | Non homogène | Non homogène |

Ces essais montrent que la formule selon l'invention (ex 4) est stable, tandis que les compositions comprenant que le polymère B (ex 5) ou que le polymère C (ex 6) ne sont pas stables.
Ainsi, l'association des polymères B et C permet de stabiliser l'émulsion.

Le sérum appliqué sur la peau s'étale facilement, en se fluidifiant, sans sensation de collant, et sans peluchage.

## Revendications

1. Composition comprenant, dans un milieu aqueux physiologiquement acceptable, un composé de formule (I) suivant : 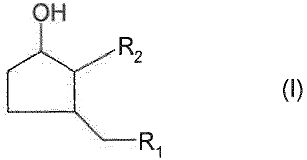 dans laquelle :
R₁ représente un radical COOR₃, R₃ désignant un atome d'hydrogène ou un radical alkyle en C₁-C₄, éventuellement substitué par un ou plusieurs groupes hydroxyle ;
R₂ représente un radical hydrocarboné, saturé ou insaturé, linéaire ayant de 1 à 18 atomes de carbones, ou ramifié ou cyclique ayant de 3 à 18 atomes de carbone ;
ainsi que leurs isomères optiques, et sels correspondants ;
un homopolymère d'un monomère à groupement sulfonique ;
et un homopolymère d'acide acrylique réticulé.

2. Composition selon la revendication 1, **caractérisé en ce que** le composé (I) est tel que R₁ désigne un radical choisi parmi -COOH, -COOMe, -COO-CH₂-CH₃, -COO-CH₂-CH(OH)-CH₂OH, -COOCH₂-CH₂-CH₂OH, -COOCH₂-CH(OH)-CH₃;
R₂ désigne un radical hydrocarboné, linéaire, saturé ou insaturé, ayant de 2 à 7 atomes de carbone.

3. Composition selon la revendication 1 ou 2, **caractérisée en ce que** le composé (I) est l'acide 3-hydroxy 2-pentyl cyclopentane acétique.

4. Composition selon l'une quelconque des revendications précédentes, **caractérisée en ce que** le composé de formule (I) est présent en une teneur allant de 1 à 10 % en poids, par rapport au poids total de la composition, de préférence de 1,5 % à 5 % en poids.

5. Composition selon l'une quelconque des revendications précédentes, **caractérisée par le fait que** le monomère à groupement sulfonique est l'acide 2-acrylamido 2-méthylpropane sulfonique.

6. Composition selon l'une quelconque des revendications précédentes, **caractérisée par le fait que** l'homopolymère de monomère à groupement sulfonique est un homopolymère réticulé et neutralisé d'acide 2-acrylamido 2-méthylpropane sulfonique.

7. Composition selon l'un quelconque des revendications précédentes, **caractérisée par le fait que** l'homopolymère de monomère à groupe sulfonique est présent en une teneur allant de 0,05 à 5 % en poids, de préférence allant de 0,1 à 5 % en poids, préférentiellement allant de 0,1 à 2 % en poids, par rapport au poids total de la composition.

8. Composition selon l'une quelconque des revendications précédentes, **caractérisée par le fait que** l'homopolymère d'acide acrylique est présent en une quantité allant de 0,01 à 5 % en poids, par rapport au poids total de la composition, et de préférence de 0,1 à 3 % en poids.

9. Composition selon l'une quelconque des revendications précédentes, **caractérisée en ce qu'**elle se présente sous la forme d'une émulsion huile-dans-eau.

10. Composition selon la revendication précédente, **caractérisée en ce qu'**elle comprend un tensioactif de type alkylpolyglycoside.

11. Composition selon la revendication 10, **caractérisée par le fait que** ledit alkylpolyglycoside est un composé de formule (II) :
R(O)(G)ₓ (II)
dans laquelle le radical R est un radical alkyle linéaire ou ramifié en C₁₂-C₂₂, x varie de 1 à 5, et G est un reste de saccharide choisi parmi glucose, dextrose, saccharose, fructose, galactose, maltose, maltotriose, lactose, cellobiose, mannose, ribose, dextrane, talose, allose, xylose, levoglucane, cellulose ou amidon.

12. Composition selon la revendication précédente, **caractérisée par le fait que** G est un reste de glucose.

13. Composition selon l'une quelconque des revendications 10 à 12, **caractérisée en ce que** le tensioactif de type alkylpolyglycoside est présent en une teneur allant de 0,1 % à 1,6 % en poids, par rapport au poids total de la composition, de préférence allant de 0,1 % à 1,5 % en poids, et préférentiellement allant de 0,1 % à 1 % en poids.

14. Composition selon l'une quelconque des revendications 10 à 13, **caractérisée en ce qu'**elle comprend un alcool gras comprenant de 10 à 30 atomes de carbone.

15. Composition selon la revendication précédente, **caractérisée en ce que** l'alcool gras est présent en une teneur allant de 0,4 % à 8 % en poids, par rapport au poids total de la composition, de préférence allant de 0,1 % à 1 % en poids, et préférentiellement allant de 0,6 % à 2 % en poids.

16. Composition selon l'une quelconque des revendications précédentes, **caractérisée en ce qu'**elle comprend une huile en une teneur allant de 0,5 % à 20 % en poids, par rapport au poids total de la composition, de préférence allant de 1 % à 15 % en poids.

17. Composition selon l'une quelconque des revendications précédentes, **caractérisée en ce qu'**elle comprend un additif cosmétique choisi parmi les émulsionnants, les gélifiants, les huiles, les cires, les conservateurs, les antioxydants, l'eau, les parfums, les charges, les filtres UV, les pigments, les fibres, les agents chélatants, les absorbeurs d'odeur, les matières colorantes.

18. Procédé de traitement cosmétique non thérapeutique des matières kératiniques comprenant l'application sur lesdites matières kératiniques d'une composition cosmétique telle que définie selon l'une quelconque des revendications précédentes.

## Patentansprüche

1. Zusammensetzung, die in einem physiologisch unbedenklichen wässrigen Medium eine Verbindung der folgenden Formel (I): 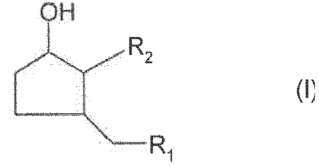 in der:
R₁ für einen COOR₃-Rest steht, wobei R₃ ein Wasserstoffatom oder einen C₁-C₄-Alkylrest, der gegebenenfalls durch einen oder mehrere Hydroxylgruppen substituiert ist, bedeutet;
R₂ für einen gesättigten oder ungesättigten Kohlenwasserstoffrest, der linear ist und 1 bis 18 Kohlenstoffatome aufweist oder verzweigt oder
cyclisch ist und 3 bis 18 Kohlenstoffatome aufweist, steht;
sowie optische Isomere und entsprechende Salze davon;
ein Homopolymer eines Monomers mit einer Sulfonsäuregruppe
und ein vernetztes Acrylsäure-Homopolymer umfasst.

2. Zusammensetzung nach Anspruch 1, **dadurch gekennzeichnet, dass** die Verbindung (I) so beschaffen ist, dass R₁ für einen aus -COOH,
- COOMe, -COO-CH₂-CH₃, -COO-CH₂-CH(OH)-CH₂OH,
- COOCH₂-CH₂-CH₂OH und -COOCH₂-CH(OH)-CH₃ ausgewählten Rest steht und
R₂ für einen gesättigten oder ungesättigten linearen Kohlenwasserstoffrest mit 2 bis 7 Kohlenstoffatomen steht.

3. Zusammensetzung nach Anspruch 1 oder 2, **dadurch gekennzeichnet, dass** es sich bei der Verbindung (I) um 3-Hydroxy-2-pentylcyclopentanessigsäure handelt.

4. Zusammensetzung nach einem der vorhergehenden Ansprüche, **dadurch gekennzeichnet, dass** die Verbindung der Formel (I) in einem Gehalt im Bereich von 1 bis 10 Gew.-%, bezogen auf das Gesamtgewicht der Zusammensetzung, vorzugsweise von 1,5 bis 5 Gew.-%, vorliegt.

5. Zusammensetzung nach einem der vorhergehenden Ansprüche, **dadurch gekennzeichnet, dass** es sich bei dem Monomer mit einer Sulfonsäuregruppe um 2-Acrylamido-2-methylpropansulfonsäure handelt.

6. Zusammensetzung nach einem der vorhergehenden Ansprüche, **dadurch gekennzeichnet, dass** es sich bei dem Homopolymer eines Monomers mit einer Sulfonsäuregruppe um ein vernetztes und neutralisiertes 2-Acrylamido-2-methylpropansulfonsäure-Homopolymer handelt.

7. Zusammensetzung nach einem der vorhergehenden Ansprüche, **dadurch gekennzeichnet, dass** das Homopolymer eines Monomers mit einer Sulfonsäuregruppe in einem Gehalt im Bereich von 0,05 bis 5 Gew.-%, vorzugsweise im Bereich von 0,1 bis 5 Gew.-%, bevorzugt im Bereich von 0,1 bis 2 Gew.-%, bezogen auf das Gesamtgewicht der Zusammensetzung, vorliegt.

8. Zusammensetzung nach einem der vorhergehenden Ansprüche, **dadurch gekennzeichnet, dass** das Acrylsäure-Homopolymer in einem Gehalt im Bereich von 0,01 bis 5 Gew.-%, bezogen auf das Gesamtgewicht der Zusammensetzung, und vorzugsweise 0,1 bis 3 Gew.-% vorliegt.

9. Zusammensetzung nach einem der vorhergehenden Ansprüche, **dadurch gekennzeichnet, dass** sie in Form einer Öl-in-Wasser-Emulsion vorliegt.

10. Zusammensetzung nach dem vorhergehenden Anspruch, **dadurch gekennzeichnet, dass** sie ein Tensid vom Alkylpolyglykosid-Typ umfasst.

11. Zusammensetzung nach Anspruch 10, **dadurch gekennzeichnet, dass** es sich bei dem Alkylpolyglykosid um eine Verbindung der Formel (II) handelt:
R(O)(G)ₓ (II)
in der der Rest R ein linearer oder verzweigter C₁₂-C₂₂-Alkylrest ist, x von 1 bis 5 variiert und G für einen Saccharidrest steht, der aus Glucose, Dextrose, Saccharose, Fructose, Galactose, Maltose, Maltotriose, Lactose, Cellobiose, Mannose, Ribose, Dextran, Talose, Allose, Xylose, Lävoglucan, Cellulose und Stärke ausgewählt ist.

12. Zusammensetzung nach dem vorhergehenden Anspruch, **dadurch gekennzeichnet, dass** G ein Glucoserest ist.

13. Zusammensetzung nach einem der Ansprüche 10 bis 12, **dadurch gekennzeichnet, dass** das Tensid vom Alkylpolyglykosid-Typ in einem Gehalt im Bereich von 0,1 bis 1,6 Gew.-%, bezogen auf das Gesamtgewicht der Zusammensetzung, vorzugsweise im Bereich von 0,1 bis 1,5 Gew.-% und bevorzugt im Bereich von 0,1 bis 1 Gew.-% vorliegt.

14. Zusammensetzung nach einem der Ansprüche 10 bis 13, **dadurch gekennzeichnet, dass** sie einen Fettalkohol mit 10 bis 30 Kohlenstoffatomen umfasst.

15. Zusammensetzung nach dem vorhergehenden Anspruch, **dadurch gekennzeichnet, dass** der Fettalkohol in einem Gehalt im Bereich von 0,4 bis 8 Gew.-%, bezogen auf das Gesamtgewicht der Zusammensetzung, vorzugsweise im Bereich von 0,1 bis 1 Gew.-% und bevorzugt im Bereich von 0,6 bis 2 Gew.-% vorliegt.

16. Zusammensetzung nach einem der vorhergehenden Ansprüche, **dadurch gekennzeichnet, dass** sie ein Öl in einem Gehalt im Bereich von 0,5 bis 20 Gew.-%, bezogen auf das Gesamtgewicht der Zusammensetzung, vorzugsweise im Bereich von 1 bis 15 Gew.-%, umfasst.

17. Zusammensetzung nach einem der vorhergehenden Ansprüche, **dadurch gekennzeichnet, dass** sie ein kosmetisches Additiv umfasst, das aus Emulgatoren, Geliermitteln, Ölen, Wachsen, Konservierungsstoffen, Antioxidantien, Wasser, Duftstoffen, Füllstoffen) UV-Filtern, Pigmenten, Fasern, Chelatbildnern, Geruchsabsorbern und Farbmitteln ausgewählt ist.

18. Verfahren zur nichttherapeutischen kosmetischen Behandlung von Keratinfasern, bei dem man auf die Keratinfasern eine kosmetische Zusammensetzung gemäß einem der vorhergehenden Ansprüche aufbringt.

## Claims

1. Composition comprising, in a physiologically acceptable aqueous medium, a compound of formula (I) below: 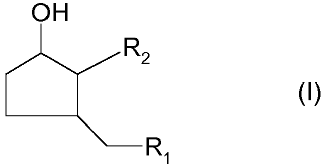 in which:
R₁ represents a radical COOR₃, R₃ denoting a hydrogen atom or a C₁-C₄ alkyl radical, optionally substituted with one or more hydroxyl groups;
R₂ represents a saturated or unsaturated linear hydrocarbon-based radical containing from 1 to 18 carbon atoms or a saturated or unsaturated branched or
cyclic hydrocarbon-based radical containing from 3 to 18 carbon atoms;
and also the optical isomers thereof, and corresponding salts;
a homopolymer of a monomer comprising a sulfonic group; and a crosslinked acrylic acid homopolymer.

2. Composition according to Claim 1, **characterized in that** the compound (I) is such that R₁ denotes a radical chosen from -COOH, -COOMe, -COO-CH₂-CH₃, -COO-CH₂-CH (OH)-CH₂OH, -COOCH₂-CH₂-CH₂OH , -COOCH₂-CH(OH)-CH₃;
R₂ denotes a saturated or unsaturated linear hydrocarbon-based radical containing from 2 to 7 carbon atoms.

3. Composition according to Claim 1 or 2, **characterized in that** the compound (I) is 3-hydroxy-2-pentylcyclopentaneacetic acid.

4. Composition according to any one of the preceding claims, **characterized in that** the compound of formula (I) is present in a content ranging from 1% to 10% by weight, relative to the total weight of the composition, and preferably from 1.5% to 5% by weight.

5. Composition according to any one of the preceding claims, **characterized in that** the monomer comprising a sulfonic group is 2-acrylamido-2-methylpropanesulfonic acid.

6. Composition according to any one of the preceding claims, **characterized in that** the homopolymer of a monomer comprising a sulfonic group is a crosslinked and neutralized homopolymer of 2-acrylamido-2-methylpropanesulfonic acid.

7. Composition according to any one of the preceding claims, **characterized in that** the homopolymer of a monomer comprising a sulfonic group is present in a content ranging from 0.05% to 5% by weight, preferably ranging from 0.1% to 5% by weight, preferentially ranging from 0.1% to 2% by weight, relative to the total weight of the composition.

8. Composition according to any one of the preceding claims, **characterized in that** the acrylic acid homopolymer is present in an amount ranging from 0.01% to 5% by weight, relative to the total weight of the composition, and preferably ranging from 0.1% to 3% by weight.

9. Composition according to any one of the preceding claims, **characterized in that** it is in the form of an oil-in-water emulsion.

10. Composition according to the preceding claim, **characterized in that** it comprises a surfactant of alkylpolyglycoside type.

11. Composition according to Claim 10, **characterized in that** said alkylpolyglycoside is a compound of formula (II) :
R(O)(G)ₓ (II)
in which the radical R is a linear or branched C₁₂-C₂₂ alkyl radical, x ranges from 1 to 5 and G is a saccharide residue chosen from glucose, dextrose, saccharose, fructose, galactose, maltose, maltotriose, lactose, cellobiose, mannose, ribose, dextran, talose, allose, xylose, levoglucan, cellulose and starch.

12. Composition according to the preceding claim, **characterized in that** G is a glucose residue.

13. Composition according to any one of Claims 10 to 12, **characterized in that** the surfactant of alkylpolyglycoside type is present in a content ranging from 0.1% to 1.6% by weight, relative to the total weight of the composition, preferably ranging from 0.1% to 1.5% by weight and preferentially ranging from 0.1% to 1% by weight.

14. Composition according to any one of Claims 10 to 13, **characterized in that** it comprises a fatty alcohol comprising from 10 to 30 carbon atoms.

15. Composition according to the preceding claim, **characterized in that** the fatty alcohol is present in a content ranging from 0.4% to 8% by weight, relative to the total weight of the composition, preferably ranging from 0.1% to 1% by weight and preferentially ranging from 0.6% to 2% by weight.

16. Composition according to any one of the preceding claims, **characterized in that** it comprises an oil in a content ranging from 0.5% to 20% by weight, relative to the total weight of the composition, preferably ranging from 1% to 15% by weight.

17. Composition according to any one of the preceding claims, **characterized in that** it comprises a cosmetic additive chosen from emulsifiers, gelling agents, oils, waxes, preservatives, antioxidants, water, fragrances, fillers, UV screens, pigments, fibers, chelating agents, odor absorbers and colorants.

18. Non-therapeutic cosmetic treatment process for keratin materials, comprising the application to said keratin materials of a cosmetic composition as defined in any one of the preceding claims.
